# EUROPEAN PATENT SPECIFICATION

(11) **EP 4 496 836 B1**
(45) Date of publication and mention of the grant of the patent: **10.12.2025**
(21) Application number: 23741437.0
(22) Date of filing: 19.06.2023
(51) Int. Cl.: A61Q 19/00, A61K 8/92, A61K 8/86, A61K 8/73, A61K 8/65, A61K 8/02, C08J 3/205, A61K 8/04, A23D 9/04, A23D 9/007

(54) **METHOD FOR PRODUCING OLEOGELS AND OLEOGELS OBTAINABLE BY THE METHOD**
VERFAHREN ZUR HERSTELLUNG VON OLEOGELEN UND NACH DIESEM VERFAHREN HERSTELLBARE OLEOGELE
PROCÉDÉ DE PRÉPARATION D'OLÉOGELS ET OLÉOGELS POUVANT ÊTRE PRÉPARÉS SELON CE PROCÉDÉ

(30) Priority: 27.06.2022 FI 20225585
(43) Date of publication of application: 29.01.2025
(73) Proprietor: Perfat Technologies Oy, 00790 Helsinki (FI)
(72) Inventor: VALOPPI, Fabio, 00790 Helsinki (FI); SALMI, Ari, 00640 Helsinki (FI); HAEGGSTRÖM, Edward, 00140 Helsinki (FI); LAIDMÄE, Ivo, 61601 Tartumaa (EE); HEINÄMÄKI, Jyrki, 51013 Tartu (EE)
(74) Representative: Laine IP Oy
(86) International application number: PCT/FI2023/050365
(87) International publication number: WO 2024/003446

(56) References cited:
- WO-A1-2012/084427
- WO-A1-2013/092024
- VALOPPI FABIO ET AL: "Formation and characterization of oleogels obtained via direct dispersion of ultrasound-enhanced electrospun nanofibers and cold milling", FOOD STRUCTURE, vol. 37, 1 July 2023 (2023-07-01), pages 100338, XP093083726, ISSN: 2213-3291, DOI: 10.1016/j.foostr.2023.100338
- BORREGO MARÍA ET AL: "Electrospun lignin-PVP nanofibers and their ability for structuring oil", INTERNATIONAL JOURNAL OF BIOLOGICAL MACROMOLECULES, ELSEVIER BV, NL, vol. 180, 16 March 2021 (2021-03-16), pages 212 - 221, XP086570905, ISSN: 0141-8130, [retrieved on 20210316], DOI: 10.1016/J.IJBIOMAC.2021.03.069
- RUBIO-VALLE JOSÉ F. ET AL: "Electrohydrodynamic Processing of PVP-Doped Kraft Lignin Micro- and Nano-Structures and Application of Electrospun Nanofiber Templates to Produce Oleogels", POLYMERS, vol. 13, no. 13, 3 July 2021 (2021-07-03), pages 2206, XP093083721, DOI: 10.3390/polym13132206

## Description

### FIELD

The present disclosure relates to methods for producing oleogels. The disclosure relates also to oleogels obtainable by the method, and use of the oleogels.

### BACKGROUND

Oleogels are semi-solid self-supporting lipid-based materials developed as substitutes for saturated and hydrogenated fats. They are composed of large volumes (> 85%) of liquid oil entrapped in a three-dimensional network of gelators.

Oleogels can be prepared using direct and indirect methods. Indirect methods are foam-, emulsion-, solvent-exchange, and aerogel-templated methods, where proteins or polysaccharides are used to prepare a scaffold in which oil is absorbed or retained. However, these methods are time consuming, expensive, and currently industrially unfeasible.

The direct method does not need specific equipment, requires little energy, and is industrially scalable. In the method crystalline molecules are used as oleogelators to directly gel the oil followed by heating and cooling steps. The oleogelators rearrange themselves during cooling to form a crystalline network. The network entraps the oil and gels the system. However, the applicability of oleogels produced by the direct method as replacements for saturated fats is hindered by storage instability, low tolerance to shear force, oxidation of essential fatty acids during oleogel production, and high digestibility, Moreover, the direct method can be applied only with molecules that are dispersible in oil and have self-assembly properties, all of which limiting the production of oleogels.

Borrego M. et al. (International Journal of Biological Macromolecules, 2021, Vol. 180, pp. 212-221) disclosed a method for producing an oleogel lubricant including slow addition of castor oil to electrospun nanostructures consisting of low-sulfonate Kraft lignin (KL) and polyvinylpyrrolidone (PVP). KL/PVP concentration in the oleogel was 10-30 wt.-%.

Rubio-Valle, J. F. et al. (Polymers, 2021, Vol. 13, No. 2206, pp. 1-15) disclosed a method for producing oleogel lubricants by dispensing eucalyptus Kraft lignin (ELK) /PVP nanostructures in castor oil at gentle agitation. Stable oleogels were formed when the EKL/PVP concentration was higher than 15 wt.-%.

WO 2013/092024 A1 relates to edible compositions comprising a fat phase wherein the fat phase is structured by a lipophilic fibrous material comprising a polymer.

WO 2012/084427 A1 relates to compositions comprising non-aqueous liquid phases (such as vegetable oil or fat, dairy oil or fat, fish oil or fat) structured by a lipophilic fibrous material comprising prolamins and/or lipophilic cellulose derivatives.

However, there still is need for further methods for producing oleogels.

### SUMMARY

Accordingly, it is an object of the present disclosure to provide a new method for producing oleogels, wherein the method comprising the following steps
a) providing a polymer nanofiber mat, wherein the polymer is edible,
b) cutting the polymer nanofiber mat into pieces, wherein average area of the pieces is 1 cm² or less, preferably 5 mm² or less,
c) adding the pieces of the polymer nanofiber mat to an edible oil to form an admixture, wherein concentration of the pieces of the polymer nanofiber mat in the admixture at least 5 wt.-%, more preferably at least 10 wt.-%, provided that when the polymer is polyethylene oxide, the concentration is at least 10 wt.-% and
d) milling the admixture at a temperature below glass transition temperature of the polymer but at 20 °C or below thereby producing the oleogel.

It is also an object of the present disclosure to provide oleogels obtainable by a method comprising the following steps
a) providing a polymer nanofiber mat, wherein the polymer is edible,
b) cutting the polymer nanofiber mat into pieces, wherein average area of the pieces is 1 cm² or less, preferably 5 mm² or less,
c) adding the pieces of the polymer nanofiber mat to an edible oil to form an admixture, wherein concentration of the polymer nanofiber mat in the admixture is at least 5 wt.-%, more preferably at least 10 wt.-%, provided that when the polymer is polyethylene oxide, the concentration is at least 10 wt.-% and
d) milling the admixture at a temperature below glass transition temperature of the polymer but at 20 °C or below thereby producing the oleogel.

It is still an object of the present disclosure to provide a new use of the oleogels obtainable by a method of the present disclosure as substitute for saturated and/or hydrogenated fat.

Exemplifying and non-limiting embodiments of the invention, both as to constructions and to methods of operation, together with additional objects and advantages thereof, are best understood from the following description of specific and exemplifying embodiments when read in connection with the accompanying drawings.

The verbs "to comprise" and "to include" are used in this document as open limitations that neither exclude nor require the existence of unrecited features. The features recited in the accompanied depending claims are mutually freely combinable unless otherwise explicitly stated. Furthermore, it is to be understood that the use of "a" or "an", i.e. a singular form, throughout this document does not exclude a plurality.

### BRIEF DESCRIPTION OF DRAWINGS

Figure 1 shows the macroscopic visual appearance (A) and SEM (B) of an ultrasonically enhanced electrospun polyethylene oxide (PEO) nanofiber mat.
Figure 2 shows visual appearance (top row) and polarized light micrographs (bottom row) for (A) 10 wt.-% PEO nanofiber mat in rapeseed oil, (B and B1) dispersion of 10 wt.-% cryo-milled PEO nanofibers (8 min milling time) in rapeseed oil, (C and C1) dispersion of 10 wt-% cryo-milled PEO nanofibers (14 min milling time) in rapeseed oil, and (D and D1) dispersion of 10 wt.-% of cryo-milled PEO powder (8 min milling time) in rapeseed oil.
Figure 3 shows the visual appearance (top row) and polarized light micrographs (bottom row) for (A and A1) 2.5 wt.-%, (B and B1) 5 wt.-%, (C and C1) 10 wt.-%, and (D and D1) 20 wt.-% PEO nanofibers in rapeseed oil. PEO nanofibers were cut in small pieces, added to oil and subsequently the dispersion was cryo-milled.
Figure 4 shows a scanning electron microscopy image of de-oiled oleogel containing 10 wt.-% PEO nanofibers.
Figure 5 shows visual appearance (top row) and polarized light micrographs (bottom row) for oleogels containing 10 wt-% PEO nanofibers in (A and A1) walnut oil, and (B and B1) flaxseed oil. PEO nanofibers were cut into small pieces, added to oil and subsequently the dispersion was cryo-milled.
Figure 6 shows polarized light micrographs of oleogels containing 10 wt.-% PEO nanofibers in rapeseed oil during heating (from 20 °C to 80 °C) and cooling (from 80 °C to 20 °C) at 5 °C/min.
Figure 7 shows thixotropic recovery of oleogels obtained through milling at room temperature of mixtures containing (A) 10 wt-% gelatin, (B) 15 wt-% gelatin, (C) 10 wt-% dextran, and (D) 5 wt-% high amylose starch nanofiber mat pieces, in rapeseed oil (RSO), walnut oil (WO), or flaxseed oil (FSO).
Figure 8 shows the microstructure of oleogels containing 10 wt-% or 15 wt.-% gelatin, and 10 wt.-% dextran electrospun nanofiber mats in rapeseed, walnut, and flaxseed oils after milling at room temperature.

### DESCRIPTION

According to one aspect the present invention concerns a method for producing oleogels. The method comprises the following steps:
a) providing a polymer nanofiber mat,
b) cutting the polymer nanofiber mat into pieces, wherein average area of the pieces is 1 cm² or less, preferably 5 mm² or less,
c) adding the pieces of the polymer nanofiber mat to an oil to form an admixture, wherein concentration of the pieces of the polymer nanofiber mat in the admixture is more than 1 wt.-%, preferably at least 5 wt.-%, more preferably at least 10 wt.-% and
d) milling the admixture at a temperature below glass transition temperature of the polymer thereby producing the oleogel.

The polymer nanofiber mat is preferably produced by electrospinning. Electrospinning (ES) is a nanotechnology that employs an electric field to pull nanofibers from a polymer solution. A Taylor cone is formed at the surface of the polymer solution extrusion, typically charged with high voltage (several -kV), and it serves as the location of the ejection of an initial fibre. The final fibre is typically formed through a drying process. The product is collected onto a charged collector by electric attraction (typically higher potential than at the polymer, e.g., ground or positive potential). Different implementations of ES exist which feature different potentials at the source polymer and target.

According to one embodiment the polymer nanofiber is produced using conventional ES, wherein fibre ejection takes place from a small polymer droplet located at an orifice, e.g., at a tip of a charged needle. The polymer fibre thus formed is guided towards the collector plate. The mat is obtained by moving the collector plate. The droplet is refilled by pumping new polymer solution to the needle tip through the needle.

According to a preferable embodiment the polymer nanofiber mat is prepared using a syringe-free electrospinning device wherein the nanofibers are generated by using ultrasound. An exemplary electrospinning device suitable for the method is disclosed in EP 3274491.

For producing the polymer nanofiber mat using an electrospinning device such as that of EP 3274491, step a) of the method comprises the following
i. providing a polymer medium,
ii. subjecting the polymer medium to an ultrasound field comprising preferably at least one of a pulse, a burst, thereby forming a protrusion on a surface of the polymer medium,
iii. applying voltage to the polymer medium thereby forming a Taylor cone on the protrusion, and ejecting a polymer fibre from tip of the Taylor cone, and
iv. guiding the polymer fibre towards the collector plate while moving the collector plate, thereby producing the polymer nanofiber mat.

According to a preferable embodiment the polymer medium is liquid. The ultrasound, preferably ultrasound burst or pulse, generates a protrusion on the top surface of the polymer medium located in the open chamber of the device. It is preferred that the ultrasound burst, or pulse is sufficiently strong that the tip of the protrusion is above the top edge of the walls of the open chamber. This ensures a stable electric field between the tip of the protrusion and the target by diminishing the effects of the walls on the electric DC-field.

The voltage is applied to the polymer medium by using an electrode located in the open chamber and within the polymer medium. The voltage is preferably between 5 kV and 50 kV. This forms a Taylor cone and thus ejection of a polymer fiber from the tip of the Taylor cone. The ejecting polymer fiber is collected onto a grounded or charged collector that is flat, conical or of any other geometric shape. The collectors are known in the art. The nanofibers of the polymer nanofiber mat have typically a diameter of 5 nm to 1000 nm or up to 2000 nm. The larger fibers are not preferred if the oleogels are for use as substitute of saturated and/or hydrogenated fat of a food product since an oleogel including particles over 20 µm may feel in mouth as grainy.

The polymer suitable for the method is preferably selected from a group consisting of polyethylene oxide, cellulose, and derivatives thereof, pectin, resistant starch, zein, agar, β-glucan, and mixtures thereof.

Further polymers suitable for the method include gelatin, dextran, high amylose starch, and hydroxypropylmethyl cellulose (HPMC).

The oil suitable for the method may be selected from a group consisting of soybean oil, canola oil, corn oil, sunflower oil, safflower oil, flaxseed oil, almond oil, peanut oil, fish oil, algal oil, palm oil, palm stearin, palm olein, palm kernel oil, high oleic soybean/canola/sunflower/safflower oils, hydrogenated palm kernel oil, hydrogenated palm stearin, fully hydrogenated soy-bean/canola/cottonseed oils, high stearic sunflower oil, enzymatically and chemically inter-esterified oils, butter oil, cocoa butter, avocado oil, almond oil, coconut oil, cottonseed oil, mineral oil, silicon oil, fluorinated oil, and mixtures thereof, preferably from rapeseed oil, walnut oil, and flaxseed oil and mixtures thereof.

According to a preferable embodiment the oil is an edible oil, and the polymer is an edible polymer.

According to another embodiment the oil is a non- edible oil, and the polymer is a non-edible polymer.

Non-limiting exemplary polymer/oil combinations are the following PEO/rapeseed oil; gelatin/rapeseed oil, dextran/rapeseed oil, starch/rapeseed oil, PEO/walnut oil, gelatin/walnut oil, dextran/walnut oil, starch/walnut oil, PEO/flaxseed oil, gelatin/flaxseed oil, dextran/flaxseed oil, and starch/flaxseed oil.

According to the method the polymer nanofiber mat is added to an oil to form an admixture. The mat is cut into pieces before admixing. Average area of the pieces of the polymer nanofiber mat is 1 cm² or less, preferably 5 mm² or less. An exemplary piece is a square of 2·2 mm². To obtain an oleogel using electrospun nanofibers mats, the diameter of the nanofibers is preferably between 100 nm and 2000 nm. The concentration of the polymer nanofiber mat in the admixture is more than 1 wt.-%, preferably at least 5 wt.-%, more preferably at least 10 wt.-%, even more preferably 10-25 wt.-%, still even more preferably 10-20 wt.-% such as 10 wt.-%. The optimal concentration is dependent on the polymer.

The method comprises milling the mixture. The milling is performed below the temperature wherein the polymer transits to a glassy state. The crystallization and glass transition temperatures are known in the art, or they can be measured using known procedures. The milling temperature may be 20 °C or lower, such as below 4 °C. The optimal milling temperature depends on the properties of the oil and the polymer.

According to an embodiment the milling comprises cryo-milling the admixture, preferably at a temperature from -180 °C to -150 °C.

It is known that some oils transit to glassy state without crystallization if cooled rapidly enough. For example, castor oil, when cooled at rates more than 5 °C/min, forms a lipid glass.

According to one embodiment the milling of step c) comprises
i. cooling the admixture from a first temperature to a second temperature by a rate of at least 5 °C/min, wherein the first temperature is 20 °C or less to a second temperature, wherein the second temperature is temperature wherein the oil crystallizes or transits to a glassy state, or below, and
ii. milling the admixture at the second temperature.

The diameter of nanofiber mat fragments obtained by milling in the oil is preferably between 10 µm and 200 µm.

According to one aspect the present disclosure concerns oleogels obtainable by the method. When the oleogels are used as substitutes for saturated and/or hydrogenated fat of food, it is essential that the oil is an edible oil, and the polymer is an edible polymer. However, there are applications wherein the oil and the polymer does not need to be edible. For example, when the oleogel is used as a lubricant, it is even preferable that the oil and the polymer are not edible.

### Experimental

### Materials

Polyethylene oxide (PEO, 900 kDa) gelatin, HPMC, Tween 20, glacial acetic acid, and formic acid were purchased from Sigma Aldrich (St. Louis, USA). High amylose starch (HYLON^{®} VII) was from Ingredion (Westchester, Illinois, United States). Rapeseed, walnut, and flaxseed oils were purchased in a local supermarket. Deionized water was using in all experiments.

### Preparation of solutions

The following solutions were prepared
(i) 4% (w/v) PEO in deionized water,
(ii) 14% (w/w) gelatin in glacial acetic acid,
(iii) 35-36% (w/v) dextran in deionized water including 1-2% (w/w) Tween 20,
(iv) 17-19% (w/w) high amylose starch in mixture of formic acid and deionized water (70:30; v/v),
(v) 2% (w/w) HPMC in deionized water.

All solutions were stirred overnight at ambient temperature prior to electrospinning.

### Nanofiber formation using ultrasound enhanced electrospinning (USES)

PEO-based nanofibers were produced using a custom-made ultrasound enhanced electrospinning (USES) device disclosed in EP 3274491. A 4% (w/w) solution of PEO in water was added to the USES device. Electrospinning was initiated by applying 8 kV voltage between the spinning solution and the collector plate positioned 15 cm above the solution. The piezoelectric transducer was driven using a 260-mV sine wave. Other ultrasound parameters were as follows: pulse repetition frequency of 100 Hz, duty cycle of 11%, cycles per burst 2970. USES was performed at 40% RH and 23 °C. PEO nanofiber mats were collected after 2 h of spinning.

A spinning solution prepared as disclosed above was added to the USES system. An acoustic fountain was generated with a pulsed sinusoidal signal with a 2.7-MHz driving frequency. Pulse repetition frequency, duty cycle, and corresponding cycle rates per burst were adjusted to obtain a stable spinning. Electrospinning was initiated by applying a 9-15-kV voltage between the spinning solution and the collector plate. The collector plate was held at a constant distance of 10-17 cm above the solution. USES was performed at 20-40% RH and 20-25 °C. The PEO, gelatin, dextran, high amylose starch, and HPMC nanofiber mats produced were collected on aluminium foil after 2 h of spinning.

### Nanofiber cryo-mill procedure

USES PEO nanofiber mats were cut into pieces, added into 10-mL milling cups with two 10-mm diameter stainless steel balls, and plunged in liquid nitrogen for 10 min. Then, nanofibers were cryo-milled for 5 min, cooled again for 10 min in liquid nitrogen, and milled for another 3 min. Total milling time was 8 min. In some samples, cooling and 3-min milling were repeated two more times after the 8-min milling cycle was completed. Total milling time in the last case was 14 min.

### Oleogel formation using USES nanofibers at room temperature

The nanofiber mats were removed from the aluminium foil and cut into pieces (not exceeding 1 cm²) with scissors, added to rapeseed oil, walnut oil, and flaxseed oil at increasing concentrations (2.5%, 5%, 10%, 15% and 20%) aiming to produce 1 g oleogel in total. The mixture was added in 10-mL stainless steel milling cups with one/two 12-mm diameter stainless steel balls. Following, the mixtures were milled using an MM400 laboratory mixer mill (Retsch, Haan, Germany) between 5 s and 8 min at around 20 °C.

### Polarized light microscopy

The microstructure of oleogels was analysed using an AxioVision polarized light microscope (Zeiss, Oberkochen, Germany) connected to an AxioCam MRm digital camera. An aliquot of sample was placed on a glass slide and a coverslip was gently pressed on top of it. Samples were analysed using a 20× objective. Images were acquired and processed using ZEN 2.6 (Zeiss). Images were recorded isothermally at room temperature or during heating from 20 °C to 80 °C at 5 °C/min and subsequent cooling from 80 °C to 20 °C at 5 °C/min using a PE120 temperature control system (Linkam Scientific Instruments Ltd., Tadworth, UK) attached to the microscope stage.

### Scanning electron microscopy (SEM)

The microstructural analysis of selected oleogels was performed using SEM as described by Bhattarai *et al.* (2022). Before imaging, the oleogels were de-oiled using isobutanol (Blake & Marangoni, 2015). Samples were gently transferred onto 25 mm diameter 0.2 µm pore-sized polycarbonate disks (Whatman, Maidstone, UK) placed on top of Whatman 1 filter paper. Isobutanol was added dropwise onto the sample surface and was allowed to dry for 48 h at room temperature before imaging. Samples were coated with Au-Pd alloy (2-nm thick layer) using a Cressington HR208 high-resolution sputter coater (Cressington Scientific Instruments, Watford, UK). Imaging was performed at RT with a Hitachi S-4800 (Hitachi High-Technologies Corporation, Tokyo, Japan) field emission scanning electron microscope.

### Differential scanning calorimetry

The thermal properties of PEO powder, oils, USES PEO nanofibers, and oleogels were analysed using a DSC823e differential scanning calorimeter (Mettler Toledo, Columbus, USA) mounted with a TSO801RO sample robot (Mettler Toledo). Samples were prepared by carefully weighing 6-9 mg in 40-µL aluminium DSC pans. Samples were kept at 20 °C for 5 min and cooled from 20 to -100 °C at 5 °C/min, then held at -100 °C for 10 min, heated to 20 °C at 5 °C/min, held at 20 °C for 10 min, and repeated the same cycle one more time. In another experiment, samples were heated from 20 °C to 100 °C at 5 °C/min. Measurements were performed under nitrogen flow (50 mL/min). An empty pan was used as a reference in the DSC cell. The peak melting temperature (Tm) and peak crystallization temperature (Tc) were taken as the minimum and maximum values of heat flow during transition, respectively. The glass transition temperature (Tg) was taken as the mid-point of the transition. Total peak enthalpy (ΔH) was calculated by integrating the melting curves. Data were processed using STARe DB V9.00 software (Mettler Toledo).

### Results

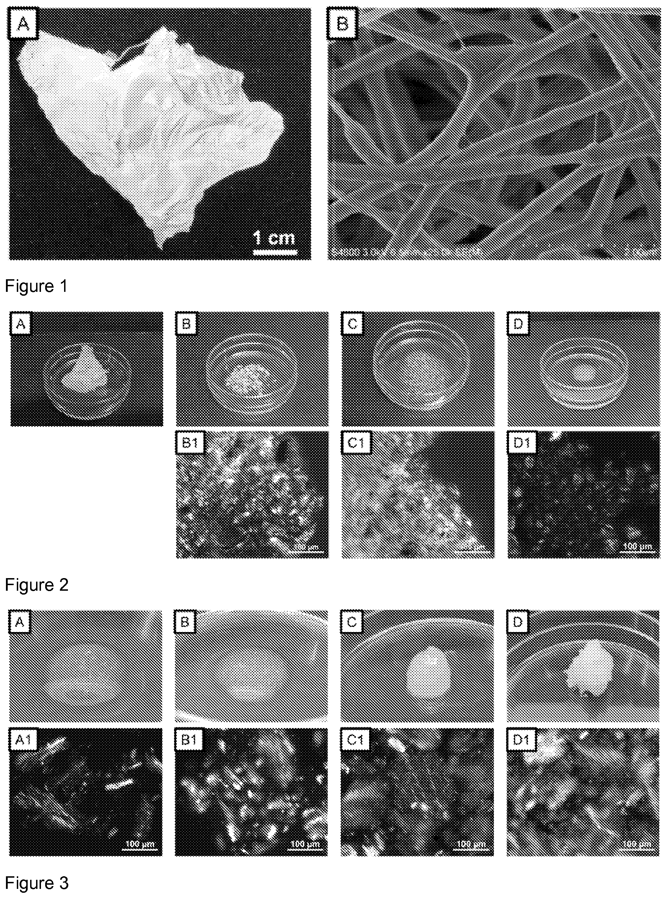

Figure 1 shows a nanofiber mat made of PEO and produced using the electrospinning device disclosed in EP 3274491. The mat appears as a light and white sheet (Fig. 1A) formed by a porous structure made of entangled PEO nanofibers with diameters ranging between 400 nm and 1000 nm (Fig. 1B). The Nanofiber mat was absorbed oil but did not form a spreadable oleogel (Fig. 2A). This might be due to the large dimensions of the mat and its interconnected internal structure. Even cutting the nanofiber mat into 2x2 mm² squares did not allow the formation of an oleogel (data not shown).

By cryo-milling the nanofiber at temperatures below its Tg (< -58.4 °C, as determined using DSC) to assure that the nanofiber structure is rigid and can be broken, and by then adding 10 wt.-% of the resulting powder to oil did not allow the formation of oleogel (Figs. 2B and 2C). Rather, a dispersion of milled fibers in oil was obtained. The inability of these cryo-milled fibers to form oleogels can be attributed to their collapsed and compact structure as shown by PL micrographs (Figs. 2B1 and 2C1). As a control, we added 10 wt-% of cryo-milled (8 min) PEO powder to oil (Fig. 2D). The dispersion was formed by compact particles of dimensions between 10 µm and 50 µm which were unable to give structure to the oil similarly to the collapsed PEO nanofibers after cryo-milling (Fig. 2D1).

On the other hand, by cutting PEO nanofiber mat in small pieces, adding it to oil and then cryo-milling the dispersion for 8 min, it was possible to obtain nanofiber fragments which showed the original porous structure of the nanofiber mat (Fig. 3A1 and 3B1). However, at low concentrations (2.5 wt.-% and 5 wt.-%) no oleogel was formed (Figs. 3A and 3B). Rather, a dispersion of diluted nanofibers was obtained.

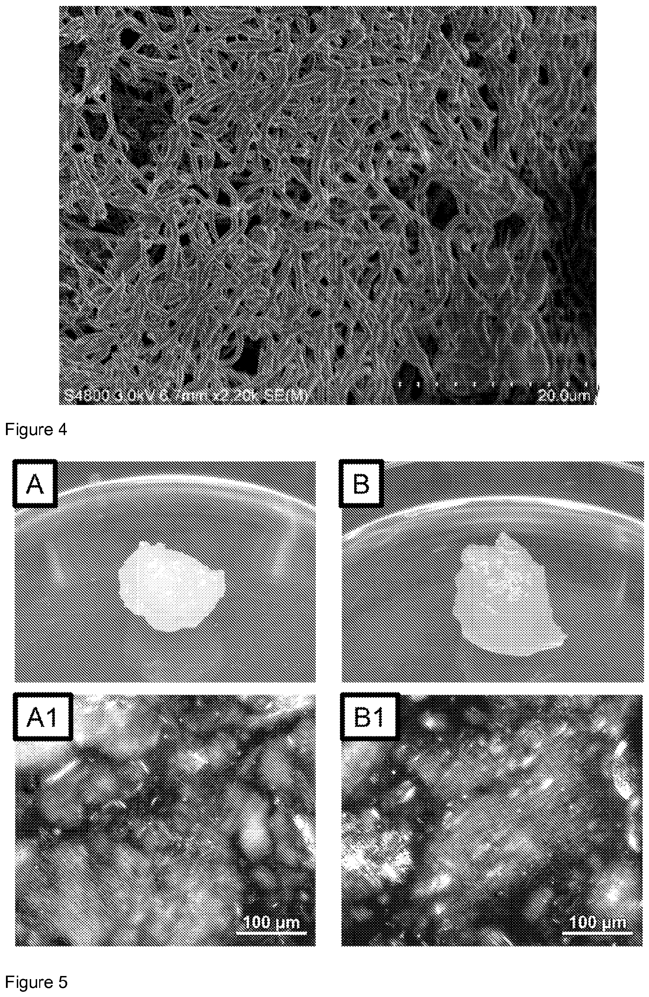

When the PEO nanofiber concentration reached 10 wt.-% (Fig. 3C and 3D) an oleogel was produced. The resulting oleogels were formed by a crowded dispersion of nanofiber mat fragments able to absorb oil in their structure (Figs. 3C1 and 3D1). PEO nanofibers can be seen in its fragments (Fig. 3C1). The retention of the nanofiber structure in the fragments after oleogel formation was confirmed by SEM after de-oiling the oleogel containing 10 wt.-% PEO nanofibers (Fig. 4). An interconnected structure of PEO nanofibers with pores can be observed like the one in Fig. 1B, meaning that the cryo-milling performed after adding PEO nanofiber mat pieces to oil protected the mat porous structure, unlike the application of cryo-milling to PEO nanofiber mat in dry conditions (Fig. 2B1 and 2C1).

To show the universality of the method, 10 wt.-% PEO nanofiber-based oleogels were prepared using walnut (Fig. 5A) and flaxseed (Fig. 5B) oils. These two oils have a higher fraction of linoleic and linolenic fatty acids compared to rapeseed oil, which mainly contains oleic acid. Both oleogels were visually similar (except for colour) to the nanofiber-based oleogel containing rapeseed oil (Fig. 3C). The microstructure is also in this case composed of fragments of nanofibers dispersed in oil where the original porous structure can be observed in the fragments (Fig. 5A1 and 5B1). The same protective mechanism by crystallized oil explained previously can take place in this case because walnut oil and flaxseed oil showed several thermal events during cooling, starting at around -10 °C and -15 °C and ending at - 80 °C and - 85 °C, with the main thermal event at -66 °C and -67 °C, respectively as determined using DSC.

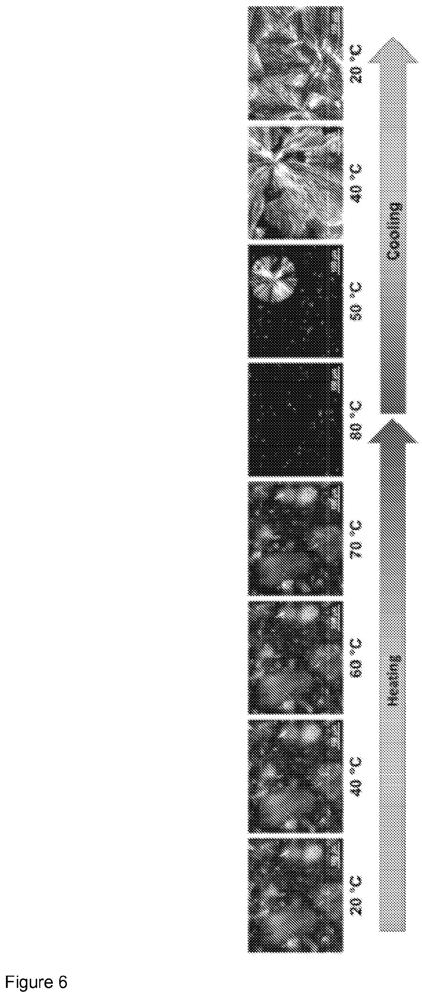

During heating the nanofiber fragments in oleogel melt and dissolve (Fig. 6). As determined by DSC, the melting temperature of PEO is at 70 °C. However, all oleogels showed a lower melting temperature comprised between 62 °C and 63.5 °C.

Upon cooling, PEO recrystallizes, however, the nanofiber porous structure is lost, and oil is expelled from the sample during PEO recrystallization, highlighting that nanofiber-based oleogels are not thermoreversible in contrast to the state-of-the-art oleogels formed with the direct method and having e.g., monoglyceride, waxes, and ethyl cellulose as oleogelators.

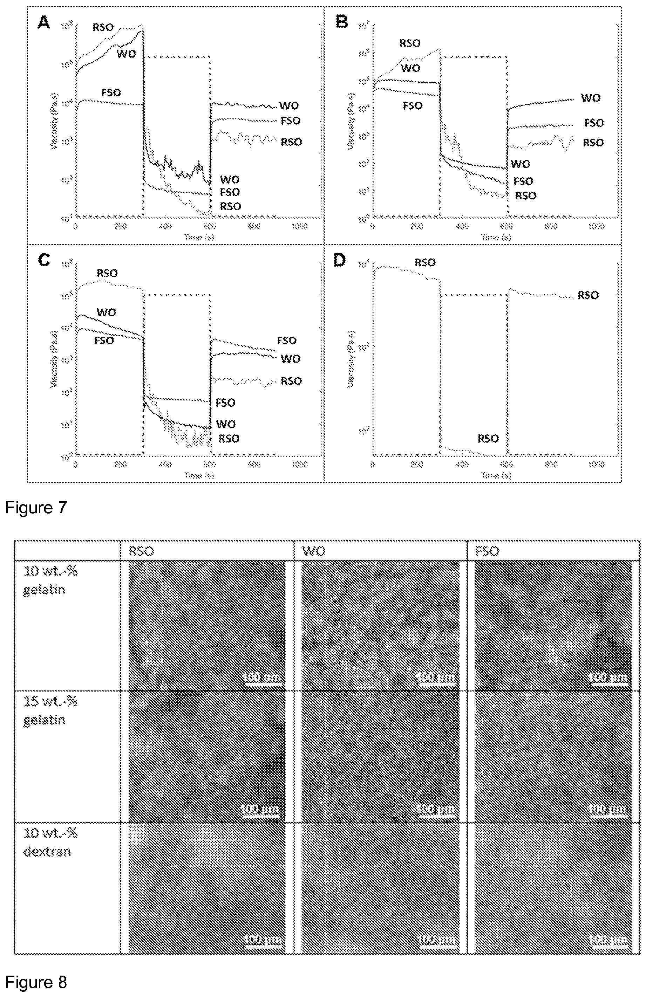

Fig. 7 shows the thixotropic recovery of oleogels made with the gelatin, dextran, and high amylose starch electrospun pieces of nanofiber mats in rapeseed, walnut, and flaxseed oils after milling at room temperature. The viscosity of the oleogels is dependent on the concentration of the biopolymer and oil used. In general, the higher the unsaturation of the oil, the lower the viscosity recorded. All samples showed thixotropic recovery which was dependent on the unsaturation level of the oil, showing better recovery for oils higher in polyunsaturated fatty acids and lower for those richer in monounsaturated fatty acids in the order FSO > WO > RSO, except for 5% starch in RSO, which showed a good thixotropic recovery.

Fig. 8 shows the microstructure of oleogels containing 10 wt.-% or 15 wt.-% gelatin, and 10 wt.-% dextran electrospun pieces of nanofiber mats in rapeseed, walnut, and flaxseed oils after milling at room temperature. Micrographs shows a jammed structure composed of nanofiber mats, their fragments, and aggregates that are dispersed in oil. The overall structure is homogeneous, and fibres were evenly distributed in the sample.

## Claims

1. A method for producing an oleogel, the method comprising the following steps
a) providing a polymer nanofiber mat, wherein the polymer is edible,
b) cutting the polymer nanofiber mat into pieces, wherein average area of the pieces is 1 cm² or less, preferably 5 mm² or less,
c) adding the pieces of the polymer nanofiber mat to an edible oil to form an admixture, wherein concentration of the pieces of the polymer nanofiber mat in the admixture is at least 5 wt.-%, more preferably at least 10 wt.-%, provided that when the polymer is polyethylene oxide, the concentration is at least 10 wt.-% and
d) milling the admixture at a temperature below glass transition temperature of the polymer but at 20 °C or below thereby producing the oleogel.

2. The method according to claim 1 wherein step a) comprises producing the polymer nanofiber mat by electrospinning.

3. The method according to claim 1 or 2 wherein step a) comprises
i. providing a polymer medium,
ii. subjecting the polymer medium to an ultrasound field thereby forming a protrusion on surface of the polymer medium,
iii. applying voltage to the polymer medium thereby forming a Taylor cone on the protrusion, and ejecting a polymer fibre from tip of the Taylor cone, and
iv. guiding the polymer fibre towards a collector plate while moving the collector plate, thereby producing the polymer nanofiber mat.

4. The method according to any one of claims 1 to 3 wherein the milling is cryo-milling.

5. The method according to any one of claims 1 to 4 wherein the polymer is selected from a group consisting of polyethylene oxide, cellulose and derivatives thereof, pectin, resistant starch, zein, agar, β-glucan, dextran, gelatin, high amylose starch, hydroxypropylmethyl cellulose (HPMC), and mixtures thereof.

6. The method according to any one of claims 1 to 5 wherein the oil is selected from a group consisting of soybean oil, canola oil, corn oil, sunflower oil, safflower oil, flaxseed oil, almond oil, peanut oil, fish oil, algal oil, palm oil, palm stearin, palm kernel oil, high oleic soybean/canola/sunflower/safflower oils, hydrogenated palm kernel oil, hydrogenated palm stearin, fully hydrogenated soy-bean/canola/cottonseed oils, high stearic sunflower oil, enzymatically and chemically inter-esterified oils, butter oil, cocoa butter, avocado oil, coconut oil, cottonseed oil, and mixtures thereof, preferably from rapeseed oil, walnut oil, and flaxseed oil and mixtures thereof.

7. An oleogel obtainable by a method according to any one of claims 1 to 6.

8. Use of the oleogel of claim 7 as a substitute of saturated and/or hydrogenated fat of a food product.

## Patentansprüche

1. Verfahren zur Herstellung eines Oleogels, wobei das Verfahren die folgenden Schritte umfasst
a) Bereitstellen einer Polymer-Nanofasermatte, wobei das Polymer essbar ist,
b) Schneiden der Polymer-Nanofasermatte in Stücke, wobei die durchschnittliche Fläche der Stücke 1 cm² oder weniger beträgt, vorzugsweise 5 mm² oder weniger,
c) Hinzufügen der Stücke der Polymer-Nanofasermatte zu einem Speiseöl, um eine Mischung zu bilden, wobei die Konzentration der Stücke der Polymer-Nanofasermatte in der Mischung mindestens 5 Gew.-%, bevorzugter mindestens 10 Gew.-% beträgt, vorausgesetzt, dass, wenn das Polymer Polyethylenoxid ist, die Konzentration mindestens 10 Gew.-% beträgt, und
d) Mahlen der Mischung bei einer Temperatur unterhalb der Glasübergangstemperatur des Polymers, jedoch bei 20°C oder darunter, wodurch das Oleogel hergestellt wird.

2. Verfahren nach Anspruch 1, wobei Schritt a) die Herstellung der Polymer-Nanofasermatte durch Elektrospinnen umfasst.

3. Verfahren nach Anspruch 1 oder 2, wobei Schritt a) Folgendes umfasst
i. Bereitstellen eines Polymermediums,
ii. Unterziehen des Polymermediums einem Ultraschallfeld, wodurch ein Vorsprung auf der Oberfläche des Polymermediums gebildet wird,
iii. Anlegen einer Spannung an das Polymermedium, wodurch ein Taylor-Kegel auf dem Vorsprung gebildet wird, und Ausstoßen einer Polymerfaser von der Spitze des Taylor-Kegels, und
iv. Führen der Polymerfaser zu einer Kollektorplatte, während die Kollektorplatte bewegt wird, wodurch die Polymer-Nanofasermatte hergestellt wird.

4. Verfahren nach einem der Ansprüche 1 bis 3, wobei das Mahlen Kryomahlen ist.

5. Verfahren nach einem der Ansprüche 1 bis 4, wobei das Polymer aus einer Gruppe ausgewählt ist, die aus Polyethylenoxid, Cellulose und Derivaten dieser, Pektin, resistenter Stärke, Zein, Agar, β-Glucan, Dextran, Gelatine, Stärke mit hohem Amylosegehalt, Hydroxypropylmethylcellulose (HPMC) und Gemische dieser besteht.

6. Verfahren nach einem der Ansprüche 1 bis 5, wobei das Öl ausgewählt ist aus der Gruppe bestehend aus Sojabohnenöl, Canolaöl, Maisöl, Sonnenblumenöl, Safloröl, Leinsamenöl, Mandelöl, Erdnussöl, Fischöl, Algenöl, Palmöl, Palmstearin, Palmkernöl, Sojabohnen-/Canola-/Sonnenblumen-/Saflorölen mit hohem Ölsäuregehalt, hydriertem Palmkernöl, hydriertem Palmstearin, vollständig hydrierten Soja-/Canola-/Baumwollsamenölen, Sonnenblumenöl mit hohem Stearinsäuregehalt, enzymatisch und chemisch umgeesterten Ölen, Butteröl, Kakaobutter, Avocadoöl, Kokosnussöl, Baumwollsamenöl und Mischungen davon, vorzugsweise aus Rapsöl, Walnussöl und Leinsamenöl und Gemische dieser.

7. Oleogel, erhältlich durch ein Verfahren nach einem der Ansprüche 1 bis 6.

8. Verwendung des Oleogels nach Anspruch 7 als Ersatz für gesättigtes und/oder hydriertes Fett eines Lebensmittelprodukts.

## Revendications

1. Procédé de production d'un oléogel, le procédé comprenant les étapes suivantes consistant à
a) fournir un tapis de nanofibres polymères, dans lequel le polymère est comestible,
b) découper le tapis de nanofibres polymères en morceaux, dans lequel la surface moyenne des morceaux est 1 cm² ou moins, de préférence 5 mm² ou moins,
c) ajouter les morceaux du tapis de nanofibres polymères à une huile comestible pour former un adjuvant, dans lequel une concentration des morceaux du tapis de nanofibres polymères dans l'adjuvant est au moins 5 % en poids, plus préférentiellement au moins 10 % en poids, à condition que, lorsque le polymère est l'oxyde de polyéthylène, la concentration soit au moins 10 % en poids et
d) broyer l'adjuvant à une température inférieure à la température de transition vitreuse du polymère, mais à 20 °C ou moins, ce qui permet de produire l'oléogel.

2. Procédé selon la revendication 1, dans lequel l'étape a) comprend la production du tapis de nanofibres polymères par électrofilature.

3. Procédé selon la revendication 1 ou 2, dans lequel l'étape a) comprend
i. la fourniture d'un milieu polymère,
ii. la soumission du milieu polymère à un champ ultrasonore, ce qui permet de former une saillie sur la surface du milieu polymère,
iii. l'application d' une tension au milieu polymère, ce qui permet de former un cône de Taylor sur la saillie, et l'éjection d'une fibre polymère de la pointe du cône de Taylor, et
iv. le guidage de la fibre polymère vers une plaque collectrice tout en déplaçant la plaque collectrice, ce qui permet de produire le tapis de nanofibres polymères.

4. Procédé selon l'une quelconque des revendications 1 à 3, dans lequel le broyage est un cryo-broyage

5. Procédé selon l'une quelconque des revendications 1 à 4, dans lequel le polymère est sélectionné dans le groupe constitué par l'oxyde de polyéthylène, la cellulose et ses dérivés, la pectine, l'amidon résistant, la zéine, l'agar, le β-glucane, le dextrane, la gélatine, l'amidon à haute teneur en amylose, l'hydroxypropylméthylcellulose (HPMC) et leurs mélanges.

6. Procédé selon l'une quelconque des revendications 1 à 5, dans lequel l'huile est sélectionnée dans le groupe constitué par l'huile de soja, l'huile de canola, l'huile de maïs, l'huile de tournesol, l'huile de carthame, l'huile de lin, l'huile d'amande, l'huile d'arachide, l'huile de poisson, l'huile d'algues, l'huile de palme, la stéarine de palme, l'huile de palmiste, les huiles de soja/canola/tournesol/carthame à haute teneur en acide oléique, l'huile de palmiste hydrogénée, la stéarine de palme hydrogénée, les huiles de soja/canola/coton entièrement hydrogénées, l'huile de tournesol à haute teneur en stéarine, les huiles interestérifiées enzymatiquement et chimiquement, l'huile de beurre, le beurre de cacao, l'huile d'avocat, l'huile de coco, l'huile de coton et leurs mélanges, de préférence parmi l'huile de colza, l'huile de noix, l'huile de lin et leurs mélanges.

7. Oléogel pouvant être obtenu par un procédé selon l'une quelconque des revendications 1 à 6.

8. Utilisation de l'oléogel selon la revendication 7 comme substitut de graisse saturée et/ou hydrogénée d'un produit alimentaire.
